# EUROPEAN PATENT APPLICATION

(11) **EP 3 733 109 A1**
(43) Date of publication of application: **04.11.2020**
(21) Application number: 18895295.6
(22) Date of filing: 30.11.2018
(51) Int. Cl.: A61B 34/35, B25J 18/06, A61B 17/062, A61B 17/28, A61B 17/3201

(54) **SURGICAL INSTRUMENT, ATTACHED DEVICE, MEDICAL TREATMENT TOOL, SURGICAL ROBOT, AND REMOTE SURGERY SYSTEM**

(30) Priority: 28.12.2017 JP 2017252805
(71) Applicant: Medicaroid Corporation, Kobe-shi, Hyogo 650-0047 (JP)
(72) Inventor: HASHIMOTO,Yasuhiko, Kobe-shi, Hyogo 650-0047 (JP)
(74) Representative: Müller Hoffmann & Partner
(86) International application number: PCT/JP2018/044224
(87) International publication number: WO 2019/130984

(57) **Abstract**

A surgical instrument (100, 110) includes: a medical treatment tool (1a, 101a) and an attachment device (6, 106, 117) to which the medical treatment tool (1a, 101a) is to be attached. The medical treatment tool (1a, 101a) includes a distal end part (20, 120) operable in multiple degrees of freedom, an elongate element (14, 114) which is a wire or a cable for operating the distal end part (20, 120), and a shaft (12, 112) that accommodates the elongate element (14, 114) and to which the distal end part (20, 120) is connected. The medical treatment tool (1a, 101a) further includes an engagement member (13, 113) connected to the elongate element on a side opposite to the distal end part (20, 120) in a longitudinal direction of the shaft (12, 112). The attachment device (6, 106, 117) further includes an engaging member (84, 184) that is to be driven by a drive source (81) and engaged with the engagement member (13, 113), and a release mechanism (73, 173) that releases an engagement between the engaging (84, 184) and the engagement member (13, 113).

## Description

### TECHNICAL FIELD

The present invention relates to a surgical instrument including a distal end part such as grasping forceps used for surgery, an attachment device and a medical treatment tool provided in the surgical instrument, and a surgical robot and a remote surgical system including at least a part thereof.

### BACKGROUND ART

In recent years, surgery support robots have been used in fields such as endoscopic surgery. A surgery support robot generally includes a patient-side apparatus including a manipulator on which an appropriate medical treatment tool is attached, and performs a surgery by remotely operating the medical treatment tool. As an example of such a medical treatment tool, for example, Patent Document 1 (International Patent Application Publication No. 2000/33755) discloses a surgical instrument that includes a distal end part, a shaft accommodating an elongate element for operating the distal end part, and an interface, such that the interface is configured to be attachable to and detachable from a robot arm.

Further, for example, Patent Document 2 (Japanese Translation of PCT International Application Publication No. 2015-521905) discloses a surgical instrument having a distal end part that is configured to be removable from a shaft.

### PRIOR ART DOCUMENTS

### PATENT LITERATURE

Patent Document 1: International Application Publication No. 2000/33755
Patent Document 2: Japanese Translation of PCT International Application Publication No. 2015-521905

### SUMMARY OF THE INVENTION

### PROBLEMS TO BE SOLVED BY THE INVENTION

However, in the surgical instrument disclosed in Patent Document 1, the distal end part, the shaft, and the interface are integrally formed. Accordingly, there is a problem that the cost for regularly purchasing the consumable surgical instrument is high, since the entire surgical instrument has to be detached from the robot arm and replaced, even if only a part of the surgical instrument needs to be replaced.

Further, in the case where the distal end part can be detached from the shaft as in the surgical instrument disclosed in Patent Document 2, the structure may be complicated and manufacturing thereof may be difficult. Further, since an attachment and detachment mechanism is included in the portion to be inserted into the body cavity, protein stains and the like are likely to remain in the attachment and detachment mechanism having a complicated structure, so that the probability of incomplete sterilization is increased.

The present invention has been made to solve the above problems, and an object thereof is to provide a surgical instrument which has a simple structure and enables replacement of a medical treatment tool at low cost, an attachment device and a medical treatment tool provided thereto, and a surgical robot and a remote system including at least a part thereof.

### MEANS FOR SOLVING THE PROBLEM

A surgical instrument according to an aspect of the present invention for achieving the above-described object includes a medical treatment tool and an attachment device to which the medical treatment tool is to be attached. The medical treatment tool includes a distal end part operable in multiple degrees of freedom, an elongate element which is a wire or a cable for operating the distal end part, and a shaft that accommodates the elongate element and to which the distal end part is connected. The medical treatment tool further includes an engagement member connected to the elongate element on a side opposite to the distal end part in a longitudinal direction of the shaft. The attachment device further includes: an engaging member that is to be driven by a drive source and engaged with the engagement member; and a release mechanism that releases an engagement between the engaging member and the engagement member.

### EFFECTS OF THE INVENTION

The present invention enables a medical treatment tool to be replaced at low cost with a simple configuration.

### BRIEF DESCRIPTION OF THE DRAWINGS

FIG. 1 is a diagram illustrating a configuration of a remote surgery system (example 1).
FIG. 2 is a diagram illustrating a configuration of a medical treatment tool (example 1).
FIG. 3 is a diagram illustrating a configuration of a distal end part of the medical treatment tool.
FIG. 4 is a diagram illustrating a configuration of an end portion of the medical treatment tool on a side opposite to the distal end part.
FIG. 5 is a diagram illustrating a configuration of the medical treatment tool and a distal end side of a robot arm.
FIG. 6 is an enlarged view illustrating a detailed configuration of an engagement member and a power conversion mechanism and an engaging member of an attachment device.
FIG. 7 is a diagram illustrating a state in which the medical treatment tool is attached on the attachment device of the robot arm.
FIG. 8 is a diagram illustrating a process of detaching the medical treatment tool from the attachment device.
FIG. 9 is a diagram illustrating a state in which a medical treatment tool is attached to an attachment device of a robot arm according to a first modification.
FIG. 10 is a diagram illustrating a process of detaching the medical treatment tool from the attachment device according to the first modification.
FIG. 11 is a diagram illustrating a state in which a medical treatment tool is attached to an attachment device of a robot arm according to a second modification.
FIG. 12 is a diagram illustrating a process of detaching the medical treatment tool from the attachment device according to the second modification.
FIG. 13 is a diagram illustrating a modified example of a configuration of a proximal end part of a medical treatment tool.
FIG. 14 is a diagram illustrating a configuration of the medical treatment tool (example 1) in which a cap is attached to the proximal end part of the medical treatment tool.
FIG. 15 is a diagram illustrating a configuration of a remote surgery system (example 2).
FIG. 16 is a diagram illustrating a configuration of a medical treatment tool (example 2).
FIG. 17 is a diagram illustrating a configuration of a remote surgery system (example 3).
FIG. 18 is a diagram illustrating a configuration of a medical treatment tool (example 3).
FIG. 19 is a diagram illustrating a detailed configuration of an end portion of a medical treatment tool on a side opposite to a distal end part, an interface, and a drive device.
FIG. 20 is a diagram illustrating a configuration of a mechanism for attaching and detaching a medical treatment tool to and from an interface.
FIG. 21 is a diagram illustrating an example of arrangement of six engagement members.

### MODE FOR CARRYING OUT THE INVENTION

### <First Embodiment>

### [Remote Surgical System]

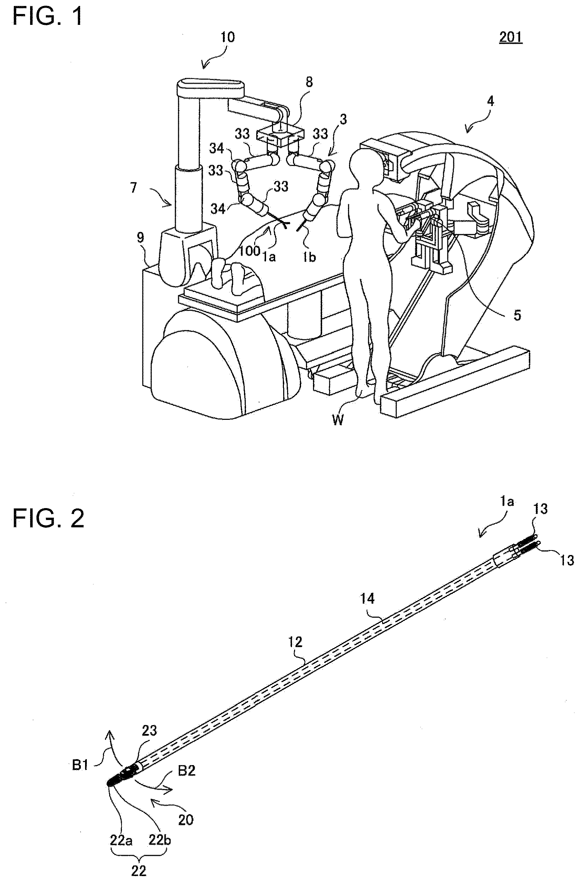

FIG. 1 is a diagram illustrating a configuration of a remote surgical system (example 1).

Referring to FIG. 1, a remote surgery system 201 includes one or more surgical instruments 100, a medical treatment tool 1a, an endoscope 1b, a remote control apparatus 4, a command input unit 5, and a patient-side apparatus (surgical robot) 10. The surgical instrument 100 includes the medical treatment tool 1a.

The medical treatment tool 1a and the endoscope 1b are attached to the robot arms 3. The robot arms 3 are electrically connected to the remote control apparatus 4 in a wired or wireless manner. The medical treatment tool 1a includes a distal end device such as grasping forceps (grasper), a needle holder (needle driver), or scissors.

When an operator W operates the command input unit 5, the command input unit 5 inputs an operation command for the patient-side apparatus 10 to the remote control apparatus 4. The remote control apparatus 4 transmits a control signal including the input operation command to the patient-side apparatus 10.

The patient-side apparatus 10 receives the control signal transmitted from the remote control apparatus 4, and operates according to the operation command included in the received control signal.

More specifically, the patient-side apparatus 10 includes a positioner 7, a platform 8 attached to a distal end of the positioner 7, the robot arms 3 attached to the platform 8, and a controller 9.

Upon receiving the control signal transmitted from the remote control apparatus 4, the controller 9 positions at least one of the platform 8 and the robot arm(s) 3 based on the operation command included in the control signal. The controller 9 also transmits the control signal to the robot arm(s) 3.

The robot arm 3 has a plurality of links 33 and a plurality of joints 34 that connect the plurality of links 33. When the robot arm 3 receives the control signal transmitted from the controller 9, the robot arm 3 operates according to the control signal to operate the corresponding medical treatment tool 1a or endoscope 1b. Thereby, the operator W can remotely operate the medical treatment tool 1a and the endoscope 1b.

The controller 9 does not necessarily have to be provided integrally with the patient-side apparatus 10, and may be provided separately from the patient-side apparatus 10.

### [Medical Treatment Tool]

### (Overall Configuration)

FIG. 2 is a diagram illustrating the configuration of the medical treatment tool (example 1).

With reference to FIG. 2, the medical treatment tool 1a includes a shaft 12 formed of an elongated rigid member, a distal end part 20 connected to a distal end of the shaft 12, a plurality of elongate elements 14 for operating the distal end part 20, and a plurality of engagement members 13 coupled to the plurality of elongate elements 14, respectively. In FIG. 2, two elongate elements 14 and two engagement members 13 are representatively illustrated.

The elongate element 14 is a rigid member such as a rod or a flexible member such as a wire or a cable, and is housed in the shaft 12.

The distal end part 20 has an end effector 22 and a wrist portion 23, and is operable with multiple degrees of freedom. The end effector 22 has a first jaw 22a and a second jaw 22b.

The first jaw 22a and the second jaw 22b are operable to move toward and away from each other, for example. The wrist portion 23 is rotatable about a rotation axis perpendicular to the Z axis, as illustrated by arrows B1 and B2.

### (Structure of Distal end part)

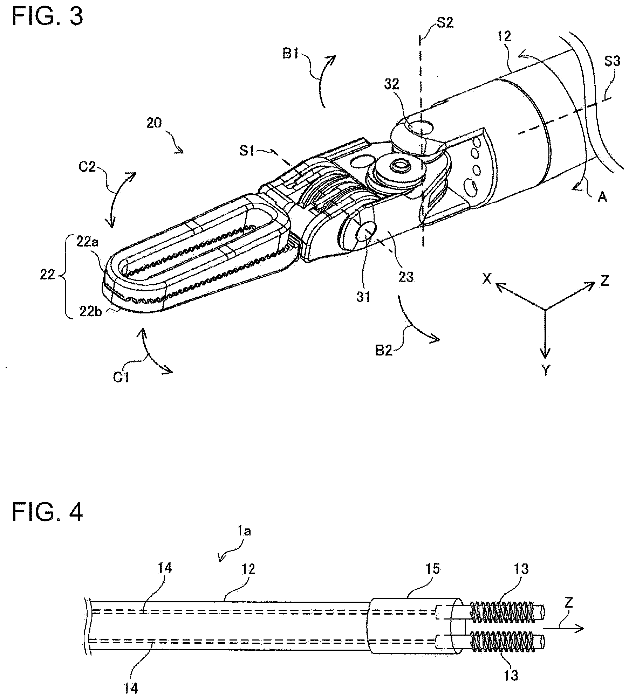

FIG. 3 is a view illustrating the configuration of the distal end part of the medical treatment tool.

With reference to FIG. 3, in addition to the end effector 22 and the wrist portion 23, the distal end part 20 further includes a first connecting portion 31 that connects the end effector 22 and the wrist portion 23 and a second connecting portion 32 that connects the wrist portion 23 and the shaft 12. The first connecting portion 31 and the second connecting portion 32 are fastening elements, such as bolts, screws, or rotational shafts, which rotatably fix the wrist portion 23 or the end effector 22.

An axis parallel to the first axis S1 defined by the first connecting portion 31 is referred to as the X axis, and an axis parallel to the second axis S2 defined by the second connecting portion 32 is referred to as the Y axis. Further, an axis parallel to the third axis S3 defined by the shaft 12 is referred to as the Z axis.

The extending direction of the first axis S1 may be slightly different from the extending direction of the first connecting portion 31. Further, the extending direction of the second axis S2 may be slightly different from the extending direction of the second connecting portion 32. Further, the extending direction of the third axis S3 may be slightly deviated from the extending direction of the shaft 12.

The first axis S1 and the second axis S2 preferably intersect with each other in a plan view from the end effector 22, that is, in a plan view seen from the negative direction to the positive direction of the Z axis. That is, it is preferable that the first axis S1, the second axis S2, and the third axis S3 are in different directions. Here, the angle formed by the first axis S1 and the second axis S2, the angle formed by the first axis S1 and the third axis S3, and the angle formed by the second axis S2 and the third axis S3 are all 90 degrees.

The wrist portion 23 is rotatable about the second axis S2 as illustrated by arrows B1 and B2. As illustrated by arrows C1 and C2, the first jaw 22a and the second jaw 22b in the end effector 22 can rotate about the first axis S1 to move toward and away from each other and also can rotate in the same direction.

More specifically, as illustrated in FIG. 3, the medical treatment tool 1a illustrated in FIG. 2 has two retractable elongate elements 14 for rotating the first jaw 22a in the direction of the arrow C2 and includes two retractable elongate elements 14 for rotating the second jaw 22b in the direction of arrow C1 and two retractable elongate elements 14 for rotating the wrist portion 23 in both the directions of the arrows B1 and B2. That is, the medical treatment tool 1a has six retractable flexible elongate elements 14 in this embodiment.

In a case where the elongate elements 14 are rigid members such as rods, one elongate element 14 can be pulled in and pushed out, so that the number of elongate elements 14 can be halved. However, since the rigid elongate elements 14 may hinder movements of bent portions of the medical treatment tool 1a, it is preferable to use flexible elongate elements 14 for a complicated structure having a high degree of freedom.

Furthermore, the operation of the first jaw 22a and the operation of the second jaw 22b can be linked. In this case, the single elongate element 14 can perform the opening/closing operation of the first jaw 22a and the second jaw 22b.

### (Structure of Elongate Element and Engagement Part)

FIG. 4 is a diagram illustrating a configuration of an end portion of the medical treatment tool on a side opposite to the distal end part. In FIG. 4, two elongate elements 14 and two engagement members 13 are representatively illustrated.

With reference to FIG. 4, the engagement member 13 is connected to an end of each elongate element 14 on an opposite side (hereinafter, may be referred to as "proximal end side") to the distal side (a side on which the distal end part 20 illustrated in FIG. 3 is located). The medical treatment tool 1a has three or more sets of the elongate elements 14 and the engagement members 13, and has six sets thereof in this embodiment.

For example, in a case where the end effector 22 of the medical treatment tool 1a is formed of a single hook-shaped member and is operated by the flexible elongate elements 14, the medical treatment tool 1a includes four pairs of the elongate elements 14 and the engagement members 13 in order to operate the end effector 22 and the wrist portion 23 by retracting operation of the elongate elements 14.

Further, the medical treatment tool 1a further includes a proximal end part 15 connected to the shaft 12 at a proximal side of the shaft 12. The shaft 12 and the proximal end part 15 communicate with each other in their internal spaces. The shaft 12 and the proximal end part 15 may be integrally formed. At least a part of the engagement member 13 is exposed from the proximal end part 15. More specifically, a groove is formed on the engagement member 13, and for example, the grooved portion of the engagement member 13 is exposed from the proximal end part 15 in the longitudinal direction of the shaft 12, and the other portion of the engagement member 13 is housed in the proximal end part 15.

The maximum dimension of the cross section of the proximal end part 15 orthogonal to the Z axis direction is formed to be slightly larger than the maximum dimension of the cross section of the shaft 12 orthogonal to the Z axis direction. Specifically, the maximum dimension of the cross section of the proximal end part 15 is a dimension that does not hinder the attachment and insertion of the proximal end part 15, and is, for example, not less than one time and not more than two times of the maximum dimension of the cross section of the shaft 12 orthogonal to the Z-axis direction. It is preferable that the maximum dimension of the cross section of the proximal end part 15 is substantially the same as that of the cross section of the shaft 12, and is preferably not more than 1.2 times that of the cross section of the shaft 12.

More specifically, the shaft 12 and the proximal end part 15 have a tubular shape, and the diameter of the proximal end part 15 is not less than one time and not more than two times the diameter of the shaft 12.

It should be noted that the shaft 12 and the proximal end part 15 are not limited to the tubular configuration, and may have a configuration having an elongated box shape, for example. Further, the shaft 12 and the proximal end part 15 are not limited to the configuration in which the cross section orthogonal to the Z-axis direction is uniform, and may have a configuration in which the outer wall has irregularities and the cross section is non-uniform.

### [Robot Arm]

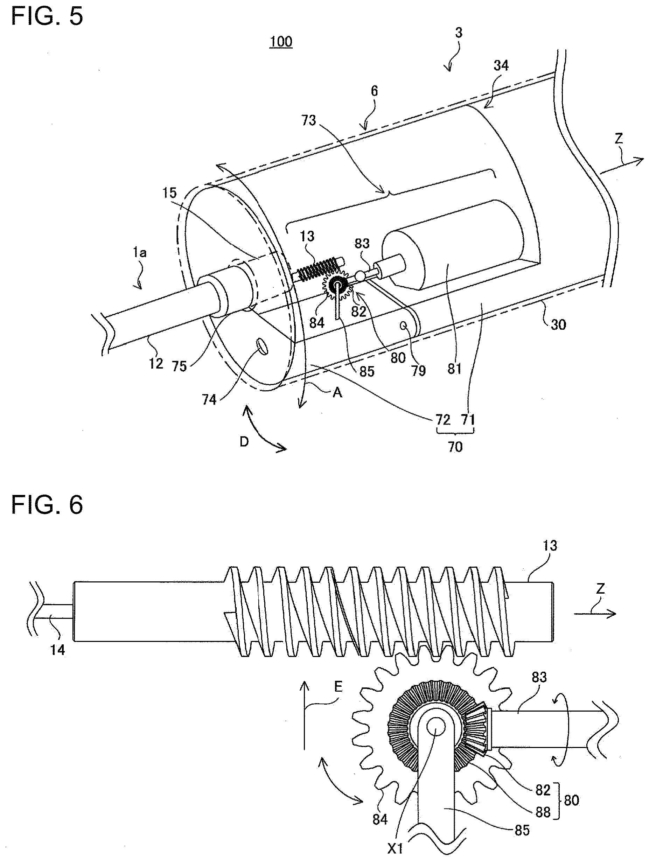

FIG. 5 is a diagram illustrating a configuration of the medical treatment tool and a distal end side of the robot arm. In FIG. 5, a part of the internal configuration of the robot arm 3 is illustrated by a solid line.

With reference to FIG. 5, the robot arm 3 includes an attachment device 6 at a distal end of a plurality of links 33, for example. That is, the attachment device 6 is provided integrally with the robot arm 3. The surgical instrument 100 includes the medical treatment tool 1a and the attachment device 6. The medical treatment tool 1a is detachably attached to the attachment device 6.

More specifically, the attachment device 6 includes a housing 70 and an attachment mechanism (release mechanism) 73 that is provided in the housing 70 and allows the medical treatment tool 1a to be attached and detached. The attachment device 6 is covered with a drape 30. In addition, in FIG. 5, the drape 30 is indicated by a two-dot chain line. The attachment mechanism 73 has a plurality of drive sources 81. The plurality of drive sources 81 operate the plurality of elongate elements 14, respectively. When the elongate elements 14 are operated, the first jaw 22a, the second jaw 22b, and the wrist portion 23 illustrated in FIG. 3 are independently driven. That is, the medical treatment tool 1a according to the embodiment of the present invention is configured to be operable with multiple degrees of freedom.

Specifically, referring again to FIG. 3, in the case where the wrist portion 23 is movable in both directions of the arrow B1 and the arrow B2, the two engagement members 13 and the two elongate elements 14 are provided such that, in the attachment mechanism 73, a first engagement member 13 is connected to a first elongate element 14 and a second engagement member 13 is connected to a second elongate element 14. A first drive source 81 operates the first engagement member 13, and a second drive source 81 operates the second engagement member 13.

Upon moving the wrist portion 23 in the direction of the arrow B1, the operator W operates the command input unit 5 to rotate the first drive source 81 in a forward direction to operate the first engagement member 13 to retract the first elongate element 14, and to rotate the second drive source 81 in a reverse direction to operate the second engagement member 13 to feed out the second elongate element 14 by the same amount.

Upon moving the wrist portion 23 in the direction of the arrow B2, the operator W operates the command input unit 5 to rotate the second drive source 81 in the forward direction to operate the second engagement member 13 to retract the second elongate element 14 and to rotate the first drive source 81 in the reverse direction to operate the first engagement member 13 to feed out the first elongate element 14 by the same amount.

In a case where the first jaw 22a and the second jaw 22b are also operated in the same operation control, four drive sources 81 are further provided, so that six drive sources 81 in total are provided.

Note that as another method for controlling the operation of the distal end part 20 of the medical treatment tool 1a, for example, a first end of the first elongate element 14 and a first end of the second elongate element 14 are attached to the wrist portion 23 so that the first elongate element 14 can be operated by the drive source 81, and a second end of the second elongate element 14 is fixed to an elastic body such as a spring.

In this case, the operator W rotates the drive source 81 in the forward direction to operate the first engagement member 13 to retract the first elongate element 14, so as to operate the wrist portion 23 in the direction of the arrow B1.

When rotating the drive source 81 in the reverse direction, the second elongate element 14 is retracted in by the elastic force of the elastic body so that the wrist portion 23 can be moved in the direction of the arrow B2. When the same operation control is performed on the first jaw 22a and the second jaw 22b, two drive sources 81 are further provided, so that three drive sources 81 in total are provided.

In a case where a rigid member such as a rod is used as the elongate element 14 as still another method for controlling the operation of the distal end part 20 of the medical treatment tool 1a, for example, one drive source 81 can retract in and feed out the elongate element 14 to move the wrist portion 23 in both directions of the arrow B1 and the arrow B2. In a case where the first jaw 22a and the second jaw 22b are operated in the same operation control, two drive sources 81 are further provided, so that three drive sources 81 in total are provided.

Referring again to FIG. 5, since the robot arm 3 includes the joint 34 on the distal end side, the attachment device 6 and the medical treatment tool 1a are operated to rotate about the Z-axis in the direction of the arrow A according to the control signal from the controller 9.

In the present embodiment, the housing 70 includes a main body portion 71 and a lower lid (lid portion) 72 as a release mechanism. The main body portion 71 includes a connecting portion 79 extending in a direction orthogonal to the longitudinal direction of the shaft 12. The lower lid 72 can be opened and closed along the direction of the arrow D by rotating about the connecting portion 79. The opening/closing operation of the lower lid 72 is performed, for example, by getting a finger of an operator caught in a recess 74 provided in the lower lid 72. In FIG. 5, the lower lid 72 is illustrated in a closed state thereof.

The attachment mechanism 73 includes: a drive source 81 housed in the housing 70, a power conversion mechanism 80 that converts rotational power of the drive source 81 into rotational power in a different direction, an engaging member 84, a connecting member 83 that connects the power conversion mechanism 80 and the drive source 81, and a support portion 85 that supports a rotational shaft of the engaging member 84.

More specifically, the housing 70 houses a plurality of sets of the drive source 81, the power conversion mechanism 80, the connecting member 83, and the engaging member 84. Further, in FIG. 5, a pair of the elongate elements 14 and the engagement member 13 are representatively illustrated. The support portion 85 supports the rotational shafts of the plurality of engaging members 84 and is fixed to the lower lid 72.

The attachment mechanism 73 may have a plurality of support portions 85 that respectively support the rotational shafts of the plurality of engagement members 84.

The drive source 81 is, for example, a servomotor, and operates under the control of the remote control apparatus 4 illustrated in FIG. 1 to operate the engaging member 84 via the connecting member 83 and the power conversion mechanism 80.

FIG. 6 is an enlarged view illustrating a detailed configuration of the engagement member and the power conversion mechanism and the engaging member in the attachment device.

With reference to FIG. 6, the power conversion mechanism 80 includes a driving side member 82 and a driven side member 88. The driving side member 82 is a bevel gear that is provided at the end of the connecting member 83 and that is connected to the drive source 81 via the connecting member 83 and that rotates in conjunction with the drive source 81, for example.

The engaging member 84 is, for example, a helical gear. On a side surface of the helical gear, a bevel gear as the driven side member 88 is provided. The engaging member 84 and the driven side member 88 are integrally formed, for example. The bevel gear of the driven side member 88 is engaged with the bevel gear of the driving side member 82. Therefore, when the drive source 81 is driven, the driving side member 82 rotates about the connecting member 83 while the driven side member 88 meshed with the driving side member 82 rotates, and thus the engaging member 84 rotates about the axis X1 orthogonal to the longitudinal direction of the connecting member 83. The engagement member 13 of the medical treatment tool 1a is engaged with the engaging member 84.

More specifically, the engagement member 13 is a worm that is engaged with the helical gear of the engaging member 84 as a worm wheel. That is, the engagement member 13 and the engaging member 84 function as a worm gear. When the engaging member 84 is rotated by driving the drive source 81, the engagement member 13, which is engaged with the engaging member 84, and the elongate element 14 connected to the engagement member 13 are moved along the Z axis.

By independently moving the plurality of elongate elements 14 along the Z axis, the corresponding first jaw 22a, second jaw 22b, and wrist portion 23 are independently driven.

Note that the engagement member 13 and the engaging member 84 may be, for example, a rack and pinion instead of the worm gear. That is, the engaging member 84 may be a spur gear, and the engagement member 13 may be a flat plate formed with a groove that is engaged with the spur gear.

Further, the engaging member 84 and the driving side member 82 are not limited to the configuration of the bevel gears. For example, the engaging member 84 may have a face gear instead of the bevel gear, and the driving side member 82 may be a spur gear or a helical gear that is engaged with the face gear.

### [Attachment and Detachment Mechanism between Medical Treatment Tool and Robot Arm]

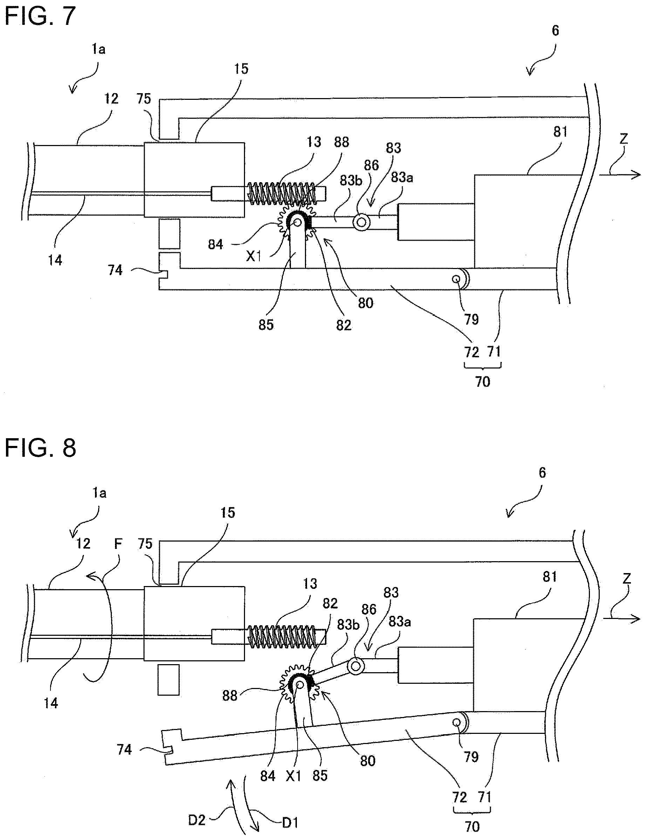

FIG. 7 is a diagram illustrating a state in which the medical treatment tool is attached to the attachment device of the robot arm. FIG. 8 is a diagram illustrating a process of detaching the medical treatment tool from the attachment device. Note that the drape 30 is not illustrated in FIGS. 7 and 8.

With reference to FIGS. 7 and 8, the engagement member 13 and the engaging member 84 are configured such that the engagement therebetween can be released. That is, the attachment mechanism 73 can release the engagement state between the engagement member 13 and the engaging member 84, and thus the medical treatment tool 1a can be attached to and detached from the attachment device 6 in the robot arm 3.

More specifically, the connecting member 83 in the attachment device 6 includes a first connecting portion 83a connected to the drive source 81, a second connecting portion 83b connected to the driving side member 82 of the power conversion mechanism 80, and a joint composed of a shaft member 86 that connects the first connecting portion 83a and the second connecting portion 83b. The first connecting portion 83a is fixed to the drive source 81. The second connecting portion 83b is rotatable around the shaft member 86.

Specifically, when detaching the medical treatment tool 1a from the attachment device 6, the operator opens the lower lid 72. That is, as illustrated in FIG. 8, the operator puts his/her finger on the recess 74 and rotates the lower lid 72 in the direction of the arrow D1 about the connecting portion 79.

At this time, the support portion 85 provided on the lower lid 72 and the engaging member 84 supported by the support portion 85 also move in the direction of the arrow D1 in conjunction with the opening operation of the lower lid 72. Also the driving side member 82 engaged with the engaging member 84 and the second connecting portion 83b connected to the driving side member 82 rotate around the shaft member 86 and thus move in the direction of the arrow D1. As a result, the engaging member 84 moves away from the engagement member 13 and the engaged state with the engagement member 13 is released. Thus, the medical treatment tool 1a can be removed through an insertion hole 75 formed in the attachment device 6.

When attaching the medical treatment tool 1a to the attachment device 6, the operator inserts the proximal end part 15 of the medical treatment tool 1a through the insertion hole 75 and closes the lower lid 72 in a state where the engaging member 13 is positioned inside the housing 70. In other words, the operator rotates the lower lid 72 about the connecting portion 79 in the direction of the arrow D2.

At this time, as illustrated in FIG. 7, the support portion 85, the second connecting portion 83b, the driving side member 82, and the engaging member 84 move, in conjunction with the closing operation of the lower lid 72, to a position where the engaging member 84 and the engagement member 13 are brought into the engaged state. Thereby, the medical treatment tool 1a can be attached to the attachment device 6.

After that, the remote control apparatus 4 performs calibration to standardize the position and posture of the distal end part 20, whereby the medical treatment tool 1a can be brought into an operable state.

With such a configuration, it is economical since the type of the surgical instrument can be changed by detaching and attaching the medical treatment tool 1a at the proximal end part 15 without replacing the entire of the medical treatment tool 1a and the attachment device 6.

Further, in a case of a structure wherein the distal end part 20 can be attached to and detached from the shaft 12, the structure becomes complicated and, after the operation, washing and sterilization work for removing blood and proteins that have been adhered in the body cavity becomes difficult. On the other hand, according to the medical treatment tool 1a, a portion of the medical treatment tool 1a closer to the distal end than the proximal end part 15 can have the same simple structure as the conventional one, and therefore the cleaning and sterilizing work on the portion can be the same as the conventional work.

### [Modification 1]

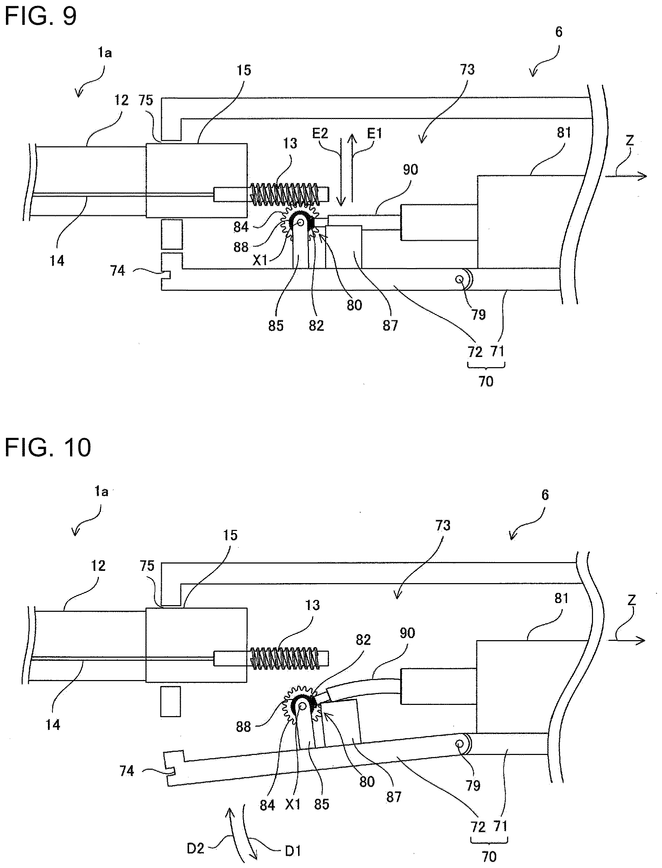

FIG. 9 is a diagram illustrating a state in which a medical treatment tool is attached to an attachment device of a robot arm according to a first modification. FIG. 10 is a diagram illustrating a process of detaching the medical treatment tool from the attachment device according to the first modification. Note that the drape 30 is not illustrated in FIGS. 9 and 10.

Referring to FIGS. 9 and 10, the attachment mechanism 73 of the robot arm 3 may have a configuration that includes a flexible member 90 provided between the drive source 81 and the driving side member 82 instead of the connecting member 83 illustrated in FIG. 7, and a support base 87. In this case, a distal side of the flexible member 90, which is, a side of the flexible member on which the engaging member 84 is located, is biased toward the direction of the arrow E2, which is orthogonal to the Z axis and away from the side on which the engagement member 13 is located. Further, in a state where the lower lid 72 is closed, the support base 87 presses the flexible member 90 in the direction of the arrow E1, which is orthogonal to the Z axis and toward the side on which the engagement member 13 is located.

When detaching the medical treatment tool 1a from the attachment device 6, the operator opens the lower lid 72, for example, with putting his / her finger on the recess 74. In other words, the operator rotates the lower lid 72 about the connecting portion 79 in the direction of the arrow D1.

At this time, the support base 87 provided on the lower lid 72 moves, and the flexible member 90, with being pressed by the support base 87, is bent in the direction of the arrow D1, which is the direction away from the side on which the engagement member 13 is located. Thus, the power conversion mechanism 80 connected to the flexible member 90 and the engaging member 84 supported by the support portion 85 move in the direction of the arrow D1. With this, the engagement between the engaging member 84 and the engagement member 13 is released, so that the medical treatment tool 1a can be detached through the insertion hole 75 formed in the attachment device 6.

When attaching the medical treatment tool 1a to the attachment device 6, the operator inserts the medical treatment tool 1a through the insertion hole 75 and closes the lid 72 in the state where the engaging member 13 is positioned in the housing 70. In other words, the operator rotates the lower lid 72 about the connecting portion 79 in the direction of the arrow D2.

At this time, the engaging member 84 supported by the support portion 85, the flexible member 90, and the power conversion mechanism 80 move so as to be in the state illustrated in FIG. 9 where the engaging member 84 and the engagement member 13 are engaged with each other. Thereby, the medical treatment tool 1a can be attached to the attachment device 6.

### [Modification 2]

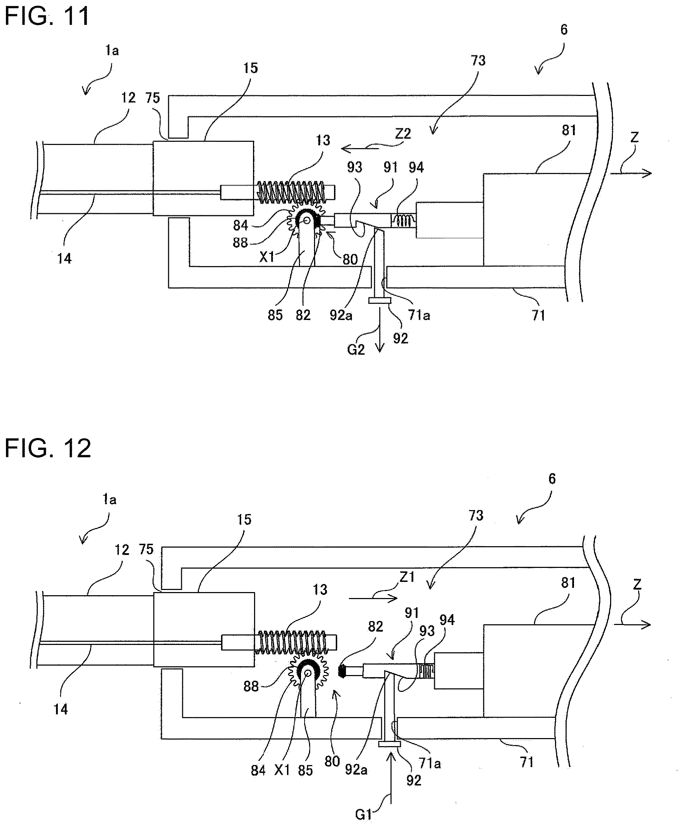

FIG. 11 is a diagram illustrating a state in which a medical treatment tool is attached to an attachment device of a robot arm according to a second modification. FIG. 12 is a diagram illustrating a process of detaching the medical treatment tool from the attachment device according to the second modification. In FIGS. 11 and 12, the drape 30 is not illustrated.

Referring to FIGS. 11 and 12, the attachment mechanism 73 of the attachment device 6 in the robot arm 3 may have a configuration that includes a connecting member 91 having a notch and a pressing member 92 instead of the connecting member 83 having the joint illustrated in FIG. 7. Further, in this case, the housing 70 may not have the lower lid 72.

The pressing member 92 is, for example, a rod-shaped member and passes through a hole 71a formed in a part of the housing 70. Further, a tip end portion 92a of the pressing member 92 located inside the housing 70 is inclined. More specifically, the tip end portion 92a is inclined such that the drive source 81 side thereof is lower and the engaging member 84 side thereof is higher.

The connecting member 91 includes a notch 93 that can be engaged with the tip end portion 92a of the pressing member 92. More specifically, the notch 93 is inclined such that the drive source 81 side thereof is shallower and the engaging member 84 side thereof is deeper. Further, the connecting member 91 includes a spring member (elastic member) 94 provided at the end portion thereof on the drive source 81 side.

As illustrated in FIG. 11, in the state where the medical treatment tool 1a is attached to the attachment device 6, the connecting member 91 is biased by the spring member 94 toward the side where the engaging member 84 is located, that is, in the direction of the arrow Z2 illustrated in FIG. 11. Further, in this state, the tip end portion 92a of the pressing member 92 is in contact with the end portion of the inclined portion of the notch 93 on the drive source 81 side, for example. The pressing member 92 is biased by a biasing member (not illustrated) toward the outside of the housing 70, that is, in the direction of the arrow G2 illustrated in FIG. 11.

When detaching the medical treatment tool 1a from the attachment device 6, the operator presses the pressing member 92, against the biasing force, toward the inside of the housing 70, that is, in the direction of the arrow G1 illustrated in FIG. 12. As a result, the pressing member 92 releases the connection between the connecting member 83 and the engaging member 84.

More specifically, at this time, the notch 93 moves the connecting member 91 in the direction away from the engaging member 84 against the biasing force of the spring member 94. That is, when the connecting member 91 is pressed by the pressing member 92, the connecting member 91 moves in the direction of the arrow Z1 in such a manner that the inclined portion of the notch 93 is moved along the inclined portion of the tip end portion 92a of the pressing member 92. With this, the driving side member 82 of the power conversion mechanism 80 connected to the connecting member 91 is released from the meshed state with the driven side member 88, and moves in the direction of the arrow Z1 which is the direction away from the engagement member 13.

As a result, the engaging member 84 rotates only with being engaged with the engagement member 13, and thus receives no stress from members other than the engagement member 13. That is, the engaging member 84 is not restricted from the operations of the members other than the engagement member 13. Therefore, by pulling out the medical treatment tool 1a from the insertion hole 75 of the housing 70 along the extending direction of the shaft 12, the medical treatment tool 1a can be detached from the attachment device 6.

Further, when attaching the medical treatment tool 1a to the attachment device 6, the operator presses the pressing member 92, against the biasing force, toward the inside of the housing 70, so that the engaging member 84 get in a state where the engagement member 84 is not restricted by the operations of the members other than engagement member 13. In such a state, the operator inserts the proximal end part 15 of the shaft 12 into the insertion hole 75 of the housing 70.

At this time, the engagement state of the driven side member 88 of the power conversion mechanism 80 with the driving side member 82 has been released. Therefore, as the shaft 12 is inserted, the engaging member 84 comes in engagement with the engagement member 13 while being rotated, for example, in the clockwise direction. Then, when the operator releases the pressing of the pressing member 92, the connecting member 91 is moved by the biasing force of the spring member 94 in the direction toward the side where the engaging member 84 is located, that is, in the direction of the arrow Z2 illustrated in FIG. 11, and thus the driving side member 82 and the driven side member 88 gets meshed with each other. As a result, the driving force of the drive source 81 can be transmitted to the engaging member 84.

### [Modification 3]

The operator may detach the medical treatment tool 1a from the attachment device 6 by operating the command input unit 5. Specifically, referring to FIG. 7, the operator operates the command input unit 5 to rotate the engaging member 84 in the counterclockwise direction, for example. When the engagement member 13 is moved to a position where the engagement with the engaging member 84 is released, the engagement member 13 is in a state where no stress is applied, and therefore the medical treatment tool 1a can be detached from the attachment device 6 by pulling out the proximal end part 15 of the medical treatment tool 1a through the insertion hole 75.

### [Modification 4]

For example, in a case where the main body portion 71 has a configuration having an upper lid (lid portion) as a release mechanism, the operator can detach the medical treatment tool 1a from the attachment device 6 by opening the upper lid and then pulling out the engagement member 13 upwardly away from the engaging member 84.

### [Modified Example of Medical Treatment Tool]

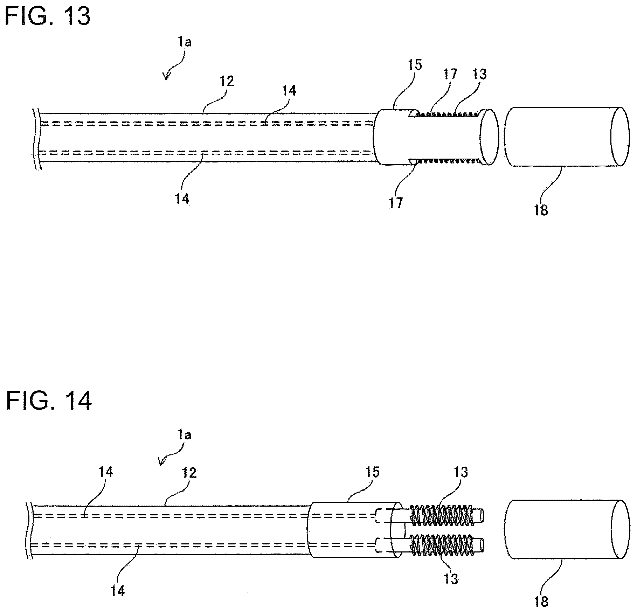

FIG. 13 is a diagram illustrating a modified example of the configuration of the proximal end part of a medical treatment tool. In FIG. 13, two sets of the elongate elements 14 and the engagement members 13 are representatively illustrated.

Referring to FIG. 13, the proximal end part 15 may be configured to accommodate the entire of the engagement member 13 in the longitudinal direction of shaft 12. In this case, the proximal end part 15 has, for example, a tubular shape in which a plurality of openings 17 are formed. At least a part of each engagement member 13 is configured to be exposed from the opening 17 in the direction orthogonal to the longitudinal direction of the shaft 12. However, the exposed portion of the engagement member 13 from the opening 17 does not project to the outside of the proximal end part 15 in the direction orthogonal to the longitudinal direction of the shaft 12.

With such a configuration, the engagement member 13 can be protected from damages and the like in a state where the medical treatment tool 1a is detached from the attachment device 6 at the time of distribution in the market or the like, and the engagement member 13 and the engaging member 84 can be engaged with each other at the time when attaching the medical treatment tool 1a to the attachment device 6 of the robot arm 3.

Further, the medical treatment tool 1a may be configured to further include a cap 18 into which the proximal end part 15 is fitted to cover the proximal end part 15 in a state where the proximal end part 15 is detached from the attachment device 6. With such a configuration, the engagement member 13 is not exposed to the outside during distribution in the market or the like, so that the engagement member 13 can be more reliably protected from damage or the like. Further, at the time when attaching the medical treatment tool 1a to the attachment device 6, the cap 18 can be removed from the proximal end part 15 to expose a part of the groove portion of the engagement member 13 so that the engagement member 13 and the engaging member 84 can be engaged.

FIG. 14 is a diagram illustrating a configuration of the medical treatment tool (example 1) in which a cap is attached to the proximal end part.

As illustrated in FIG. 13, the cap 18 is adopted not only for the configuration in which the proximal end part 15 accommodates the entire of the engagement member 13 in the longitudinal direction of the shaft 12, but also for a configuration in which at least a part of the engagement member 13 in the longitudinal direction of the shaft 12 is exposed from the proximal end part 15 as illustrated in FIG. 14.

Next, other embodiments of the present invention will be described with reference to the drawings. It should be noted that the same or equivalent parts in the drawings are designated by the same reference numerals and the description thereof will not be repeated.

### <Second Embodiment>

In the remote surgery system 201 according to the above-described first embodiment, the medical treatment tool 1a includes the shaft 12 formed of a rigid member. On the other hand, in the remote surgery system 202 according to a second embodiment, a medical treatment tool 101a includes a shaft 112 formed of a flexible member.

### [Remote Surgery System]

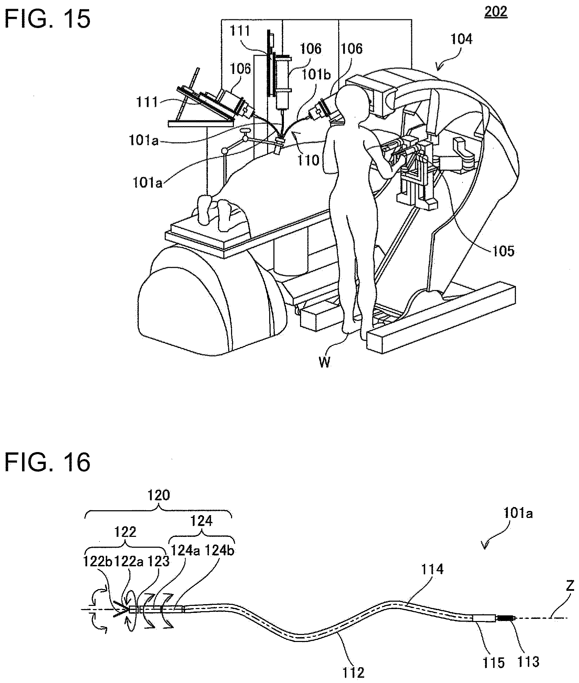

FIG. 15 is a diagram illustrating a configuration of the remote surgery system (example 2).

Referring to FIG. 15, the remote surgery system 202 includes one or more surgical instruments 110, an endoscope 101b, a remote control apparatus 104, a command input unit 105, and a plurality of drive devices (surgical robots) 106. In the present embodiment, the drive device 106 is an example of an attachment device. The surgical instrument 110 includes the medical treatment tool 101a.

The medical treatment tool 101a and the endoscope 101b are attached to the drive device 106. The drive device 106 is mounted on a support base 111, and is electrically connected to the remote control apparatus 104. The support base 111 has a known structure and supports the drive device 106 so that the drive device 106 can be moved along the longitudinal direction of the drive device 106 and can be rotated about an axis along the longitudinal direction. Like the medical treatment tool 1a illustrated in FIG. 1, the medical treatment tool 101a includes a distal end device such as a grasping forceps (grasper), a needle holder (needle driver), or scissors.

When the operator W operates the command input unit 105, the command input unit 105 gives an operation command to the remote control apparatus 104. Upon receiving the operation command from the command input unit 105, the remote control apparatus 104 transmits a control signal including the operation command to the drive device 106.

The drive device 106 has the configuration same as or similar to that of the attachment device 6 of the above-described first embodiment. Upon receiving the control signal transmitted from the remote control apparatus 104, the drive device 106 operates according to the control signal to operate the corresponding medical treatment tool 101a or endoscope 101b. This allows the operator W to remotely operate the medical treatment tool 101a and the endoscope 101b.

### [Medical Treatment Tool]

FIG. 16 is a diagram illustrating a configuration of the medical treatment tool (example 2).

Referring to FIG. 16, the medical treatment tool 101a includes the shaft 112 formed of an elongated flexible member and having flexibility, a distal end part 120 connected to a distal end of the shaft 112, a plurality of elongate elements 114 to operate the distal end part 120, a plurality of engagement members 113 respectively coupled to the plurality of elongate elements 114, and a proximal end part 115 that is coupled to the shaft 112 at the opposite side of the distal end part 120 of the shaft 112. In FIG. 15, one of the elongate elements 114 and one of the engagement members 113 are representatively illustrated.

The distal end part 120 includes an end effector 122 and an articulated portion 124. The end effector 122 includes a first jaw 122a, a second jaw 122b, and a wrist portion 123. The articulated portion 124 includes a first articulated portion 124a and a second articulated portion 124b.

The wrist portion 123 has a first end in the longitudinal direction of thereof to which the first jaw 122a and the second jaw 122b are connected, and a second end in the longitudinal direction of thereof to which the first articulated portion 124a is connected. The second articulated portion 124b is connected to an end portion of the first articulated portion 124a opposite to the wrist portion 123, and the flexible shaft 112 is connected to an end portion of the second articulated portion 124b opposite to the first articulated portion 124a.

The wrist portion 123 is rotatable about an axis extending in the longitudinal direction of the wrist portion 123. The first jaw 122a and the second jaw 122b are operable to move toward and away from each other, for example. Each of the first articulated portion 124a and the second articulated portion 124b is rotatable about an axis extending in the longitudinal direction thereof.

One ends of the elongate elements 114 are fixed to the first jaw 122a, the second jaw 122b, the wrist portion 123, the first articulated portion 124a, and the second articulated portion 124b, respectively. Each of the elongate elements 114 is a flexible member such as a wire or a cable, and is housed in the shaft 112.

The configuration of the end portion of the medical treatment tool 101a opposite to the distal end part 120 is the same as or similar to that of the medical treatment tool 1a illustrated in FIG. 4 and has a proximal end part 115. In addition, in the present embodiment, the longitudinal direction of the shaft 12 is assumed to be the same as the longitudinal direction of the proximal end part 115. As the elongate elements 114 are operated, the first jaw 122a, the second jaw 122b, the wrist portion 123, the first articulated portion 124a, and the second articulated portion 124b are independently driven.

That is, the medical treatment tool 101a according to the second embodiment of the present invention is configured to be operable in multiple degrees of freedom, specifically, seven degrees of freedom, as indicated by the arrows in FIG. 16.

Specifically, the first ends of the two elongate elements 114 are fixed to the first articulated portion 124a and the second articulated portion 124b, respectively. When the second end of one of the elongate elements 114 is pulled in, one of the first articulated portion 124a and the second articulated portion 124b that is fixed to the one elongate element 114 is bent. On the other hand, when the second end of the other elongate element 114 is pulled in, the other of the first articulated portion 124a and the second articulated portion 124b that is fixed to the other elongate element 114 is bent.

Therefore, the first articulated portion 124a and the second articulated portion 124b can be bent independently of each other, and the articulated portions 24 can be bent into complicated shapes such as a S-shaped curve.

As with the first embodiment, the first jaw 122a and the second jaw 122b can be operated independently of each other by performing a pulling operation using two elongate elements 114 for each of the jaws.

Thus, in the present embodiment, for example, six sets of the elongate elements 114 and the engagement members 113 are also provided. The wrist portion 123 can be rotated by using, for example, a torque transmission tube.

Note that the medical treatment tool 101a may be configured to be operable in three to six degrees of freedom, for example, by having a configuration in which the first jaw 122a and the second jaw 122b are not operated independently but are operated in conjunction with each other, by omitting one of the first and second articulated portions 124a and 124b, by limiting at least one of the sliding movement and the rotating movement by the drive device 106, or the like.

The attachment and detachment mechanism or method between the medical treatment tool 101a and the drive device 106 according to the second embodiment may adapt any one of the first to fourth modifications of the first embodiment described above.

The other configurations and operations are the same as or similar to the configurations and operations described in the first embodiment, and therefore detailed descriptions thereof will not be repeated here.

### <Third Embodiment>

In the remote surgery system 201 according to the above-described first embodiment, the medical treatment tool 1a is attachable to and detachable from the attachment device 6, which is a part of the robot arm 3. Further, in the remote surgery system 202 according to the second embodiment, the medical treatment tool 101a is configured to be attachable to and detachable from the drive device 106 which constitutes a surgical robot and is an example of an attachment device.

To the contrary, in a remote surgery system 203 according to a third embodiment, the robot arm 3 or a drive device 116 does not include an attachment mechanism, but an interface 117 includes the attachment mechanism. That is, the medical treatment tool 1a is configured to be attachable to and detachable from the interface 117 serving as the attachment device, and the interface 117 is further configured to be attachable to and detachable from the robot arm 3 or the drive device 116.

### [Remote Surgery System]

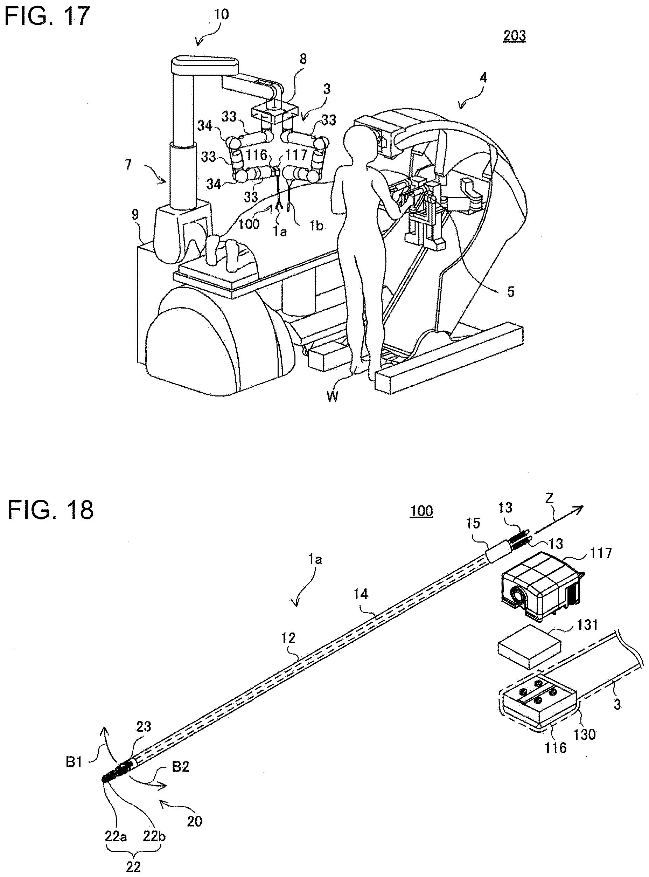

FIG. 17 is a diagram illustrating the configuration of the remote surgery system (Example 3).

With reference to FIG. 17, the remote surgery system 203 further includes the interface 117 serving as the attachment device, in comparison with the remote surgery system 201 illustrated in FIG. 1. The medical treatment tool 1a is attached to the drive device 116 via the interface 117. Further, the interface 117 is detachably attached to the drive device 116 via a drape 130 covering the robot arm 3 and a sterile adapter 131 engaged with the interface 117. Note that, in FIG. 17, the drape 130 is indicated by a two-dot chain line. The drive device 116 accommodating the drive source is provided at the distal end of the robot arm 3.

### [Medical Treatment Tool]

FIG. 18 is a diagram illustrating a configuration of the medical treatment tool (Example 3). FIG. 18 illustrates a state in which the medical treatment tool 1a is detached from the interface 117, and the interface 117 is detached from the drive device 116. Note that, in FIG. 18, the drape 130 and the sterile adapter 131 are not illustrated.

With reference to FIG. 18, the medical treatment tool 1a has the configuration same as or similar to the medical treatment tool 1a according to the first embodiment illustrated in FIG. 2.

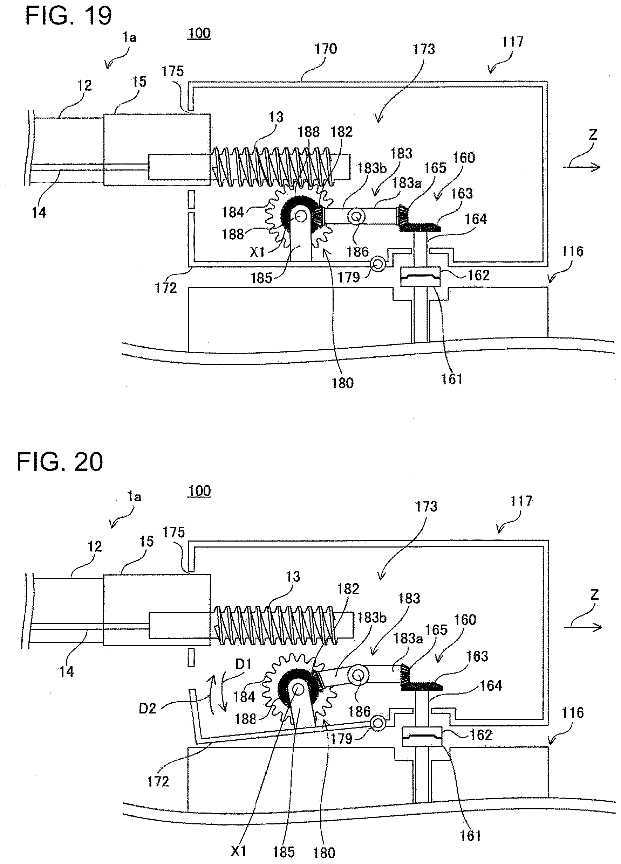

FIG. 19 is a diagram illustrating a detailed configuration of a drive device, an interface, and an end portion of the medical treatment tool on the side opposite to the distal end part. In FIG. 19, the drape 130 and the sterile adapter 131 are not illustrated.

With reference to FIG. 19, an engagement member 13 is connected to an end portion of each elongate element 14 on the side opposite to the distal end part 20, similarly to the medical treatment tool 1a illustrated in FIG. 7. Further, the medical treatment tool 1a further includes a proximal end part 15 connected to the shaft 12 on the side of the shaft 12 opposite to the distal end part 20. Internal spaces of the shaft 12 and the proximal end part 15 are communicated with each other. The shaft 12 and the proximal end part 15 may be integrally formed. Further, at least a part of the engagement member 13 is exposed from the proximal end part 15 in the longitudinal direction of the shaft 12.

The drive device 116 includes a plurality of transmission members 161, and a plurality of drive sources (not illustrated) that operate the plurality of transmission members 161, respectively.

Specifically, the drive device 116 includes two sets of the transmission members 161 and the drive sources for each of the first jaw 22a, the second jaw 22b, and the wrist portion 23 illustrated in FIG. 1, which means that the drive device 116 includes six sets thereof in total. In FIG. 19, one of the transmission members 161 is representatively illustrated.

The interface 117 includes a transmission member 162 that engages with the transmission member 161 of the drive device 116, a housing 170 provided with a lower lid (lid portion) 172 as a release mechanism, and an attachment mechanism (release mechanism) 173 provided in the housing 170. The housing 170 is attachable to and detachable from the drive device 116.

The attachment mechanism 173 includes a first power conversion mechanism 160, a second power conversion mechanism 180, a connecting member 183 that connects the first power conversion mechanism 160 and the second power conversion mechanism 180, an engaging member 184, and a support portion 185.

The first power conversion mechanism 160 includes a first driving side member 163 and a first driven side member 165, and the first driving side member 163 and the first driven side member 165 are bevel gears.

The second power conversion mechanism 180 includes a second driving side member 182 and a second driven side member 188, and the second driving side member 182 and the second driven side member 188 are bevel gears.

The engaging member 184 is, for example, a helical gear. On a side surface of the helical gear, the second driven side member 188 of the second power conversion mechanism 180, which is a bevel gear, is provided. The first driven side member 165 of the first power conversion mechanism 160 is connected to the second driving side member 182 of the second power conversion mechanism 180 via the connecting member 183.

The interface 117 further includes a transmission shaft 164 for transmitting the power from the transmission member 162 to the first power conversion mechanism 160.

The connecting member 183 includes a first connecting portion 183a connected to the first driven side member 165 of the first power conversion mechanism 160 and a second connecting portion 183b connected to the second driving side member 182 of the second power conversion mechanism 180, and a shaft member 186 that connects the first connecting portion 183a and the second connecting portion 183b. The first connecting portion 183a is fixed to the first driven side member 165 of the first power conversion mechanism 160. The second connecting portion 183b is rotatable about the shaft member 186.

The support portion 185 supports the rotation shaft of each of the plurality of engagement members 184 and is fixed to the lower lid 172 of the housing 170. Note that in FIG. 19, one of plural sets is representatively illustrated. Further, in FIG. 19, a pair of the elongate element 14 and the engagement member 13 is representatively illustrated.

The transmission member 161 in the drive device 116 has a shape having a convex portion, and the transmission member 162 in the interface 117 has a shape having a concave portion into which the convex portion of the transmission member 161 can be fit.

The engagement member 13, the engaging member 184, the second power conversion mechanism 180, the connecting member 183, and the support portion 185 have the configurations same as those of the engagement member 13, the engaging member 84, the power conversion mechanism 80, and the connecting member 83 and the support portion 85 illustrated in FIG. 7.

The drive source in the drive device 116 is operated according to the control signal from the remote control apparatus 4, to rotate the transmission member 161. The transmission member 161 transmits the power from the drive source to the transmission member 162. As a result, the transmission member 162 and the first driving side member 163 of the first power conversion mechanism 160 connected to the transmission member 162 rotate.

Along with the rotation of the first driving side member 163, the first driven side member 165 of the first power conversion mechanism 160 meshed with the first driving side member 163 and the second driving side member 182 of the second power conversion mechanism 180 connected to the first driven side member 165 rotate about the rotation axes parallel to the Z axis.

Along with the rotation of the second driving side member 182, the second driven side member 188 of the second power conversion mechanism 180 meshed with the second driving side member 182 rotates, and at the same time, the engaging member 184 rotates. With this, the engagement member 13 engaged with the engaging member 184 and the elongate element 14 connected to the engagement member 13 move along the Z axis.

By independently moving the elongate elements 14 along the Z axis, the corresponding first jaw 22a, second jaw 22b, and wrist portion 23 are independently driven.

### [Attachment and Detachment Mechanism between Medical Treatment Tool and Interface]

FIG. 20 is a diagram illustrating a configuration of the attachment and detachment mechanism between the medical treatment tool and the interface.

With reference to FIG. 20, the medical treatment tool 1a is attachable to and detachable from the interface 117. Specifically, when detaching the proximal end part 15 of the medical treatment tool 1a from the interface 117, the operator opens the lower lid 172. In other words, the operator rotates the lower lid 172 in the direction of the arrow D1 about the connecting portion 179.

At this time, the support portion 185 provided on the lower lid 172 moves, and the engaging member 184 supported by the support portion 185 moves in the direction of the arrow D1. Further, the second power conversion mechanism 180 engaged with the engaging member 184 and the second connecting portion 183b connected to the second driving side member 182 of the second power conversion mechanism 180 rotate about the shaft member 186 and move in the direction of the arrow D1. As a result, the engaging member 184 is separated from the engagement member 13 so that the engagement between the engagement member 184 and the engagement member 13 is released. Therefore, the medical treatment tool 1a can be detached from the interface 117, by pulling out the proximal end part 15 from the insertion hole 175.

On the other hand, when attaching the medical treatment tool 1a to the interface 117, the operator inserts the proximal end part 15 from the insertion hole 175 so as to position the engagement member 13 inside the interface 117 and then closes the lower lid 172. In other words, the operator rotates the lower lid 172 about the connecting portion 179 in the direction of the arrow D2.

At this time, the support portion 185, the connecting member 183, the second power conversion mechanism 180, and the engaging member 184 move so as to be in the state illustrated in FIG. 19, and the engaging member 184 and the engagement member 13 engage with each other. With this, the medical treatment tool 1a can be attached to the interface 117.

With such a configuration, it is possible to replace only the parts that need to be replaced without replacing the entire of the medical treatment tool 1a and the interface 117. For example, when the surgical instrument 100 needs to be replaced due to elongation of the elongate element 14, only the portion of the medical treatment tool 1a that is closer to the distal end than the proximal end part 15 can be replaced while the interface 117 can be continuously used. Therefore, it is economical.

Further, the surgical instrument 100 composed of the medical treatment tool 1a and the interface 117 is attached to the patient-side apparatus 10 by means of the configuration same as the conventional one. Thus, it is also economical in that the patient-side apparatus 10 having the same configuration as the conventional one can be used.

The other configurations and operations are the same as or similar to the configurations and operations described in the first and second embodiments, and therefore detailed description will not be repeated here.

Note that it is also possible to appropriately combine the features of the remote surgery systems 201, 202, and 203 according to the first, second third embodiments. For example, as the attachment and detachment mechanism or method of the medical treatment tool 1a and the interface 117 according to the third embodiment, the attachment and detachment mechanism or method described in any one of the first to fourth modifications of the first embodiment may be adopted.

Further, in each of the embodiments, only a representative set of the elongate elements 14, 114 and the engagement members 13, 113 is illustrated. For example, only two sets are illustrated in the FIG. 4. In a case where the number of the sets is increased, the structure of the attachment mechanisms 73, 173 may become complicated. Therefore, with the following configuration, it is possible to avoid the structure of the attachment mechanisms 73 and 173 from becoming complicated.

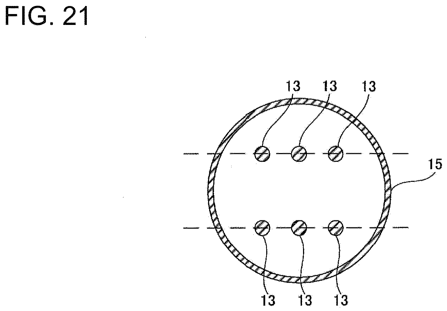

FIG. 21 is a diagram illustrating an example of arrangement of six engagement members. FIG. 21 illustrates a cross section of the proximal end part 15 of the medical treatment tool 1a orthogonal to the longitudinal direction thereof.

With reference to FIG. 21, for example, in the case where the distal end part 20 of the medical treatment tool 1a is operated by the retracting operation using the flexible elongate elements 14 as described above, six of the drive sources 81 and six sets of the elongate elements 14 and the engagement members 13 are provided in the housing 70.

The six sets are arranged in such a manner that two linear rows connecting three sets are lined up. In this case, the six drive sources 81 are also arranged in such a manner that two linear rows connecting three drive sources 81 are lined up.

In such a configuration that adopts the method for attaching and detaching the medical treatment tool 1a by opening and closing the lid as described above, the housing 70 is provided with an upper lid and a lower lid as a release mechanism, for example.

To the contrary, in such a configuration that adopts the method for attaching and detaching the medical treatment tool 1a using the pressing member 92 as described above, a pressing member located above the housing 70 and a pressing member located below the housing 70 are provided, for example.

Further, it is also possible to appropriately combine the features of the first embodiment, each of the modifications of the first embodiment, the second embodiment, and the third embodiment.

For example, a surgical system may be configured to adopt both of the method for attaching and detaching the medical treatment tool 1a by opening and closing the lower lid 72 according to the first and second modifications of the first embodiment, and the method for attaching and detaching the medical treatment tool 1a without the lower lid 72 according to the third modification of the first embodiment. In this case, even if a problem occurs in one of the methods for attaching and detaching the medical treatment tool 1a, the medical treatment tool 1a can be attached and detached by the other method, so that the resistance to failure can be improved.

Further, the configurations of the distal end parts 20 and 120 described above are examples, and any configuration can be adopted as long as the attaching and detaching method(s) described above can be used.

Further, the power conversion mechanisms 80, 180 above-described and the coupling mechanism using the connecting member 83 for power transmission above-described are examples, and a mechanism employing an additional member or a mechanism having an equivalent function may be used.

In addition, it should be considered that the above-described embodiments are exemplifications in all respects and are not restrictive. The scope of the present invention is indicated by the claims rather than the above description, and is intended to include meanings equivalent to the claims and all modifications within the scope.

### EXPLANATION OF REFERENCE NUMERALS

- 1a, 101a: Medical treatment tool
- 3: Robot arm
- 4, 104: Remote control apparatus
- 6: Attachment device
- 106, 116: Drive device
- 12, 112: Shaft
- 13, 113: Engagement member
- 14, 114: Elongate element
- 15, 115: Proximal end part
- 18: Cap
- 20, 120: Distal end part
- 30, 130: Drape
- 73, 173: Attachment mechanism (Release mechanism)
- 81: Drive source
- 83, 183: Connecting member
- 84, 184: Engaging member
- 85, 185: Support portion
- 90: Flexible member
- 100, 110: Surgical instrument
- 117: Interface (Attachment device)
- 131: Sterile adapter
- 161: Transmission member
- 162: Transmission member
- 201, 202, 203: Remote surgery system

## Claims

1. A surgical instrument comprising:
a medical treatment tool including a distal end part operable in multiple degrees of freedom, an elongate element which is a wire or a cable for operating the distal end part, and a shaft that accommodates the elongate element and to which the distal end part is connected; and
an attachment device to which the medical treatment tool is to be attached, wherein
the medical treatment tool further includes an engagement member connected to the elongate element on a side opposite to the distal end part in a longitudinal direction of the shaft, and
the attachment device includes an engaging member that is to be driven by a drive source and engaged with the engagement member, and a release mechanism that releases an engagement between the engaging member and the engagement member.

2. The surgical instrument according to claim 1, wherein
the attachment device further includes a housing that accommodates the engaging member, wherein the housing includes a lid portion to open and close the housing, and the release mechanism includes the lid portion.

3. The surgical instrument according to claim 2, wherein
the attachment device further includes a support portion that supports the engaging member thereof,
the support portion is provided to the lid portion, and
the release mechanism is configured, in conjunction with an opening operation of the lid portion, to move the support portion to a position where the engagement between the engaging member and the engagement member is released.

4. The surgical instrument according to claim 3, wherein
the release mechanism is configured, in conjunction with a closing operation of the lid portion, to move the support portion to a position where the engaging member and the engagement member are brought into the engagement.

5. The surgical instrument according to any one of claims 1 to 4, further comprising
a connecting member connected to the engaging member for transmitting a driving force from the drive source to the engaging member, wherein
the release mechanism comprises the connecting member that is composed of a flexible member or a member including a joint.

6. The surgical instrument according to claim 1, further comprising:
a housing that accommodates the engaging member; and
a connecting member connected to the engaging member for transmitting a driving force from the drive source to the engaging member, wherein
the release mechanism includes a pressing member that releases the connection between the connecting member and the engaging member by being pressed from the outside of the housing.

7. The surgical instrument according to claim 6, wherein
the release mechanism further comprises:
an elastic member that biases the connecting member toward a side where the engaging member is located; and
a notch that is provided at the connecting member and moves, by being pressed by the pressing member, the connecting member in a direction away from the engaging member against a biasing force of the elastic member.

8. The surgical instrument according to any one of claims 2 to 7, wherein
the housing further accommodates the drive source.

9. The surgical instrument according to any one of claims 2 to 8, wherein
the housing is attachable to and detachable from a drive device that houses the drive source.

10. The surgical instrument according to any one of claims 1 to 9, wherein
the engaging member is a worm wheel, and the engagement member is a worm.

11. The surgical instrument according to claim 10, wherein
the worm wheel is a helical gear or a spur gear.

12. An attachment device to which a medical treatment tool is to be attached, wherein the medical treatment tool comprises: a distal end part operable in multiple degrees of freedom; an elongate element which is a wire or a cable for operating the distal end part; a shaft that accommodates the elongate element and to which the distal end part is connected; and an engagement member connected to the elongate element on a side opposite to the distal end part in a longitudinal direction of the shaft, the attachment device comprising:
an engaging member that is to be driven by a drive source and engaged with an engagement member; and
a release mechanism that releases an engagement between the engaging member and the engagement member.

13. A medical treatment tool to be attached to the attachment device according to claim 12.

14. The medical treatment tool according to claim 13, further comprising:
a proximal end part that is provided on a side of the shaft opposite to the distal end part and which is a part where the medical treatment tool is attached to the attachment device, wherein
a maximum dimension of a cross section, orthogonal to the longitudinal direction of the shaft, of the proximal end part is not less than one time and not more than two times of a maximum dimension of a cross section of the shaft.

15. The medical treatment tool according to claim 14, wherein
at least a part of the engagement member in the longitudinal direction of the shaft is exposed from the proximal end part.

16. The medical treatment tool according to claim 14, wherein
the proximal end part accommodates an entire of the engagement member in the longitudinal direction of the shaft, and
at least a part of the engagement member in a direction orthogonal to the longitudinal direction of the shaft is exposed from the proximal end part.

17. The medical treatment tool according to claim 15 or 16, further comprising
a cap that covers the exposed portion of the engagement member and into which the proximal end part is fitted.

18. A surgical robot comprising:
a robot arm comprising a plurality of links and a plurality of joints connecting the plurality of links; and
the attachment device according to claim 12, wherein
the attachment device is provided integrally with the robot arm.

19. A surgical robot comprising:
a robot arm comprising a plurality of links and a plurality of joints connecting the plurality of links; and
the attachment device according to claim 12, wherein
the robot arm includes a drive device that accommodates the drive source,
the attachment device is detachably attached to the drive device via a drape that covers the robot arm and a sterile adapter that engages with the attachment device.

20. A remote surgery system comprising:
the surgical robot according to claim 18 or 19; and
a remote control apparatus that remotely operates the robot arm in a wireless or wired manner.
